# EUROPEAN PATENT APPLICATION

(11) **EP 3 211 087 A1**
(43) Date of publication of application: **30.08.2017**
(21) Application number: 15852335.7
(22) Date of filing: 03.09.2015
(51) Int. Cl.: C12Q 1/02, C12N 1/15, C12N 1/19, C12N 1/21, C12N 5/10, C12N 15/09, G01N 33/15, G01N 33/50, G01N 33/68

(54) **METHOD FOR SCREENING COMPOUND SPECIFICALLY INHIBITING FORMATION OF CAVEOLAE IN CANCER CELLS, SCREENING KIT, VECTOR AND TRANSFORMANT TO BE USED IN KIT, AND METHOD FOR SELECTING PATIENT ADAPTABLE FOR MOLECULAR-TARGETING DRUG**

(30) Priority: 24.10.2014 JP 2014217313
(71) Applicant: National University Corporation Nagoya University, Aichi 464-8601 (JP)
(72) Inventor: TAKAHASHI Takashi, Nagoya-shi Aichi 464-8601 (JP); YAMAGUCHI Tomoya, Nagoya-shi Aichi 464-8601 (JP)
(74) Representative: Heyerhoff Geiger & Partner Patentanwälte PartGmbB
(86) International application number: PCT/JP2015/075127
(87) International publication number: WO 2016/063637

(57) **Abstract**

Provided is a method for screening a compound, said compound being capable of specifically inhibiting the formation of caveolae in cancer cells and comprehensively inhibiting various RTK activities only by the single compound.

A screening method that is the method for screening a compound capable of specifically inhibiting the formation of caveolae in cancer cells, said screening method being characterized by comprising a step for contacting test compounds with a system capable of detecting the inhibition of the binding of Cavin-1 and CAV1, and a step for selecting a compound having an activity of inhibiting the binding of Cavin-1 and CAV1.

## Description

### BACKGROUND OF THE INVENTION

### 1. Field of the Invention

The present invention relates to a method for screening a compound specifically suppressing the formation of caveolae of cancer cells, a screening kit, a vector and transformant to be used in the kit, and a method for selecting a patient for whom a molecularly targeted drug is indicated.

### 2. Description of the Related Art

Lung cancer is a major cause of death due to cancer in economically well-developed countries, and pulmonary adenocarcinoma is the most frequent histological subtype among these cancers.

The present inventors previously reported that the persistent expression of TTF-1 (thyroid transcription factor-1), which is a lineage-specific transcription factor necessary for branching morphogenesis and physiological functions in the lung, is closely tied to pulmonary adenocarcinoma (see Non-patent Document 1). Three other groups also arrived at the same conclusion through a genome-wide search for local genomic abnormalities in pulmonary adenocarcinoma that TTF-1 is a lineage-survival oncogene (see Non-patent Documents 2-4). Since TTF-1 is essential to surfactant protein production and to physiological function in the normal adult lung, the identification of downstream molecules involved in the lineage-specific survival signal of TTF-1 is necessary for the development of new therapeutic methods.

As a result of attempts to identify such downstream molecules, the present inventors succeeded in identifying the ROR1 (receptor tyrosine kinase-like orphan receptor 1) gene as a gene whose expression is induced by TTF-1. In addition, it was clarified that silencing this ROR1 gene can inhibit the proliferation of specific cancer cells (Patent Document 1).

When the present inventors further elucidated the mechanism by which ROR1 suppresses the proliferation of cancer cells, it was clarified that it is possible to not only induce the apoptosis of cancer cells but also to suppress the survival-promoting signal of cancer cells transmitted by receptor tyrosine kinases (the term receptor tyrosine kinase is sometimes abbreviated as RTK hereinafter) such as EGFR and MET using ROR1 as a target, and that ROR1 can serve as an important target for the development of cancer cell proliferation inhibitors (Patent Document 2, Non-Patent Document 5).

### [Prior Art Documents]

### [Patent Documents]

[Patent Document 1] WO2010/008069
[Patent Document 2] WO2012/090939

### [Non-patent Documents]

[Non-patent Documents 1] Tanaka.H et al.,Cancer Res,2007,Vol.67, p6007∼6011
[Non-patent Documents 2] Kendall.J et al., Proc Natl Acad Sci USA,2007, Vol.104, p16663∼16668
[Non-patent Documents 3] Weir,BA et al., Nature, 2007, Vol.450, p893∼898
[Non-patent Documents 4] Kwei,KA et al., Oncogene,2008, Vol.27, p3635∼3640
[Non-patent Documents 5] Yamaguchi.T et al., Cancer Cell,2012, Vol.21, p348∼361
[Non-patent Documents 6] Parton.RG et al., Nat Rev Mol Cell Biol,2013, Vol.14, p98∼112
[Non-patent Documents 7] Sunaga.N et al.,Cancer Res, 2004,Vol.64, p4277∼4285
[Non-patent Documents 8] Liu.L et al., J Biol Chem, 2008, Vol.283, p4314∼4322
[Non-patent Documents 9] Liu.L et al., Cell Metab, 2008, Vol.8, p310∼317
[Non-patent Documents 10] Hill.MM et al., Cell, 2008, Vol.132, p113∼124

### SUMMARY OF THE INVENTION

### PROBLEMS TO BE SOLVED BY THE INVENTION

However, as described in the above Patent Document 2, many of the molecularly targeted drugs developed thus far are inhibitors of kinase activity. The abovementioned inhibitor of kinase activity is a compound that suppresses the activation of RTK involved in the proliferation and survival of human cancers, including lung cancer. The problem, however, when an inhibitor of kinase activity is administered to a patient is that cancer cells acquire resistance to the inhibitor of kinase activity in question by giving rise to mutations that impart resistance to the kinase gene during treatment or by the survival signal of the cancer cells creating bypass pathways dependent on other receptors.

When one of the cancer cell receptors such as EGFR or MET is used as the molecular target as described in the above Patent Document 2, signals from other receptors cannot be blocked, and an adequate effect is not obtained. Combined use of inhibitors targeting each receptor are being attempted. The problem, however, is that additional bypass pathways may be created even if multiple inhibitors are used in combination, and there is a risk of expanding side effects due to administration of multiple inhibitors.

From the above, normal cells and cancer cells can make use of various survival signals to survive themselves, but these survival signals are transmitted through many RTKs present on the cell membrane. In previous research, molecularly targeted drugs targeting individual RTKs in cancer cells (such as Iressa targeting EGFR) have been developed, and attempts have been made, as mentioned above, to use a combination of inhibitors against multiple RTKs. Furthermore, given the appearance of bypass pathways through different RTKs and the fact that these RTKs play an important role in transmitting survival signals in normal cells as well, side effects become a very serious problem, and overall these attempts have not contributed to a very significant improvement in the survival rate of cancer patients. Based on this background, a single cancer cell-specific compound that can suppress the activity of various RTKs without targeting individual RTKs in cancer cells and that does not affect normal cells is needed. However, there are no molecules or inhibitors thus far that suppress the activity of many RTKs in a cancer cell-specific manner, and it is not currently clear which control mechanisms of cancer cells should be the focus to solve these problems.

The present invention is an invention intended to solve the above problems. It was newly discovered as a result of in-depth studies that (1) by focusing on ROR1, which is a protein specific to cancer cells, and suppressing the binding of ROR1 and Cavin-1 or ROR1 and Caveolin 1 (sometimes referred to hereinafter as CAV1), the binding of Cavin-1 and CAV1 is suppressed; as a result, the formation of caveolae of cancer cells can be suppressed specifically by suppressing the stability of CAV1, and (2) given based on (1) above that it is clear that the formation of caveolae of cancer cells can be suppressed specifically, there is a possibility that the formation of caveolae of cancer cells can be specifically suppressed by screening for compounds that target Cavin-1 and the like that affect the formation of caveolae and, as a result, that the activity of various RTKs can be inhibited all at once by a single compound.

The present inventors also identified the binding sites of ROR1 binding to Cavin-1 and CAV1. Thus, it was newly discovered that compounds of (2) above can be screened simply by preparing a transformant that expresses the binding site and Cavin-1 and/or CAV1 using a vector containing DNA of the binding site in question and a vector containing DNA of Cavin-1 and/or CAV1. Since the expression of ROR1 and IGF-IR plays an important role in stabilization of the caveolae of cancer cells, the present inventors also newly discovered that a patient for whom a molecularly target drug obtained by the above screening is indicated can be selected by studying combinations of the expression of ROR1, IGF-IR, Cavin-1, and CAV1.

Specifically, the purpose of the present invention is to provide a method for screening a compound specifically suppressing the formation of caveolae of cancer cells, a screening kit, a vector and transformant to be used in the kit, and a method for selecting a patient for whom a molecularly targeted drug is indicated.

### MEANS FOR SOLVING THE ABOVEMENTIONED PROBLEMS

The invention, as shown below, relates to a method for screening a compound specifically suppressing the formation of caveolae of cancer cells, a screening kit, a vector and transformant to be used in the kit, and a method for selecting a patient for whom a molecularly targeted drug is indicated.

(1) A method for screening a compound specifically suppressing the formation of caveolae of cancer cells,
   wherein the method for screening includes
   a step for bringing a test compound into contact with a system capable of detecting suppression of the binding of Cavin-1 and CAV1, and
   a step for selecting a compound having activity to suppress the binding of Cavin-1 and CAV1.
(2) The method for screening according to (1) above, wherein the system capable of detecting suppression of the binding of Cavin-1 and CAV1 is a system capable of detecting suppression of the function of Cavin-1.
(3) The method for screening according to (2) above, wherein the system capable of detecting suppression of the function of Cavin-1 is a system capable of detecting suppression of the binding of ROR1 and Cavin-1 or a system capable of detecting suppression of the binding of IGF-IR and Cavin-1.
(4) The method for screening according to (1) above, wherein the system capable of detecting suppression of the binding of Cavin-1 and CAV1 is a system capable of detecting suppression of the function of CAV1.
(5) The method for screening according to (4) above, wherein the system capable of detecting suppression of the function of CAV1 is a system capable of detecting suppression of the binding of ROR1 and CAV1 or a system capable of detecting suppression of the binding of IGF-IR and CAV1.
(6) A transformant into which have been introduced
   a recombinant vector into which a nucleic acid that encodes an amino acid sequence including at least the amino acid region 564 to 746 within the amino acid sequence 428 to 937 of the intracellular region of human ROR1 has been inserted, and
   a recombinant vector into which a nucleic acid that encodes the amino acid sequence of human Cavin-1 has been inserted.
(7) A screening kit for a compound suppressing the binding of ROR1 and Cavin-1 including the transformant according to (6) above.
(8) A transformant into which have been introduced
   a recombinant vector into which a nucleic acid that encodes an amino acid sequence including at least the amino acid region 853 to 876 within the amino acid sequence 428 to 937 of the intracellular region of human ROR1 has been inserted, and
   a recombinant vector into which a nucleic acid that encodes the amino acid sequence of human CAV1 has been inserted.
(9) A screening kit for a compound suppressing the binding of ROR1 and CAV1 including the transformant according to (8) above.
(10) A transformant into which have been introduced
   a recombinant vector into which at least a nucleic acid that encodes an amino acid sequence including the amino acid region 564 to 746 and a nucleic acid that encodes an amino acid sequence of the amino acid region 853 to 876 within the amino acid sequence 428 to 937 of the intracellular region of human ROR1 have been inserted,
   a recombinant vector into which a nucleic acid that encodes the amino acid sequence of human Cavin-1 has been inserted, and
   a recombinant vector into which a nucleic acid that encodes the amino acid sequence of human CAV1 has been inserted.
(11) A screening kit for a compound suppressing the binding of ROR1 and Cavin-1 and/or a compound suppressing the binding of ROR1 and CAV1 including the transformant according to (10) above.
(12) A recombinant vector into which at least a nucleic acid that encodes an amino acid sequence including the amino acid region 564 to 746 and/or a nucleic acid that encodes an amino acid sequence including the amino acid region 853 to 876 within the amino acid sequence 428 to 937 of the intracellular region of human ROR1 have been inserted.
(13) A method for selecting a patient for whom a molecularly targeted drug is indicated, comprising
   a step for acquiring cancer cells from the subject and
   a step for measuring the expression of at least one selected from ROR1 and Cavin-1, ROR1 and CAV1, IGF-IR and Cavin-1, IGF-IR and CAV1, Cavin-1 and CAV1, Cavin-1, or CAV1 in the cancer cells.

### EFFECT OF THE INVENTION

By a method for screening a compound that specifically suppresses caveolae formation in cancer cells, there is a possibility of discovering a compound that suppresses cancer cells specifically without affecting normal cells.

Compounds that suppress caveolae formation in cancer cells specifically can also be screened easily by preparing a transformant that expresses the binding site and Cavin-1 and/or CAV1 using a vector containing DNA of a binding site of ROR1 that binds to Cavin-1 and/or CAV1 and a vector containing DNA of Cavin-1 and/or CAV1, and preparing a screening kit for using this transformant.

Furthermore, since the expression of ROR1 plays an important role in the stabilization of caveolae, and patients for whom a molecularly target drug obtained by the above screening is indicated can be selected by studying combinations of expression of ROR1, IGF-IR, Cavin-1, and CAV1, companion diagnostics can be prescribed.

### BRIEF DESCRIPTION OF THE DRAWINGS

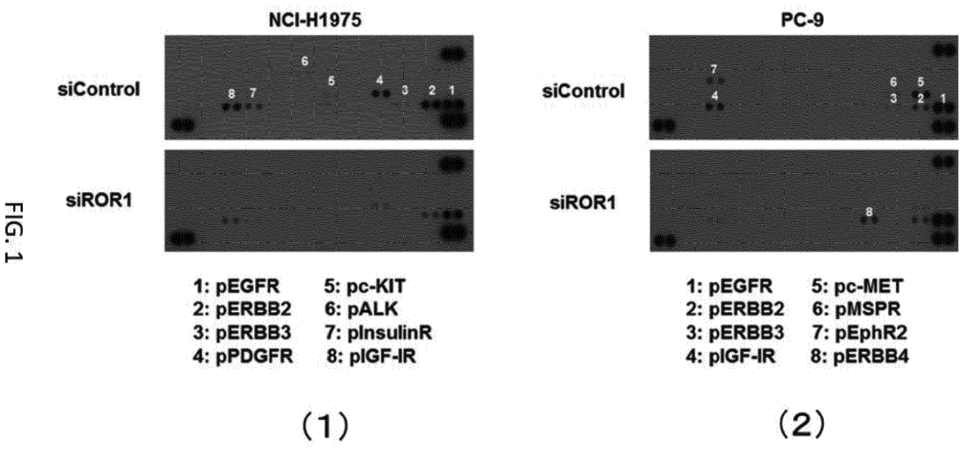

FIG. 1 is a photograph substituted for a drawing showing that silencing of ROR1 lowers the phosphorylation reaction (activation) of various RTKs, using NCI-H1975 as the pulmonary adenocarcinoma cell line in FIG. 1(1) and using PC-9 as the pulmonary adenocarcinoma cell line in FIG. 1(2).

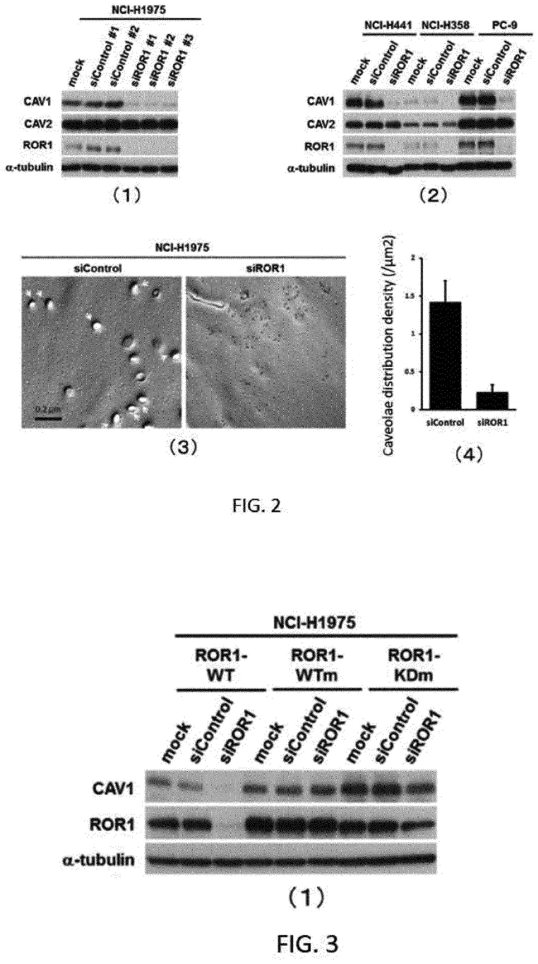

FIG. 2(1) is a photograph substituted for a drawing that is a photograph of a Western blot showing downregulation of CAV1 by silencing of ROR1 when NCI-H1975 was used as the pulmonary adenocarcinoma cell line. FIG. 2(2) is a photograph substituted for a drawing that is a photograph of a Western blot showing downregulation of CAV1 by silencing of ROR1 using NCI-H441, NCI-H358, and PC-9 as pulmonary adenocarcinoma cell lines. FIG. 2(3) is a photograph substituted for a drawing and is a photograph of electron microscope analysis of the NCI-H1975 cell membrane using rapid freezing/freeze-fracture replica labeling. FIG. 2(4) is a graph showing the results of measurement of the number of normal caveolae found on a random region of the cell membrane in NCI-H1975 treated by siControl and siROR1.
FIG. 3 is a photograph substituted for a drawing and is a photograph of a Western blot showing that the kinase activity of ROR1 is not necessary for downregulation of CAV1 by silencing of ROR1.

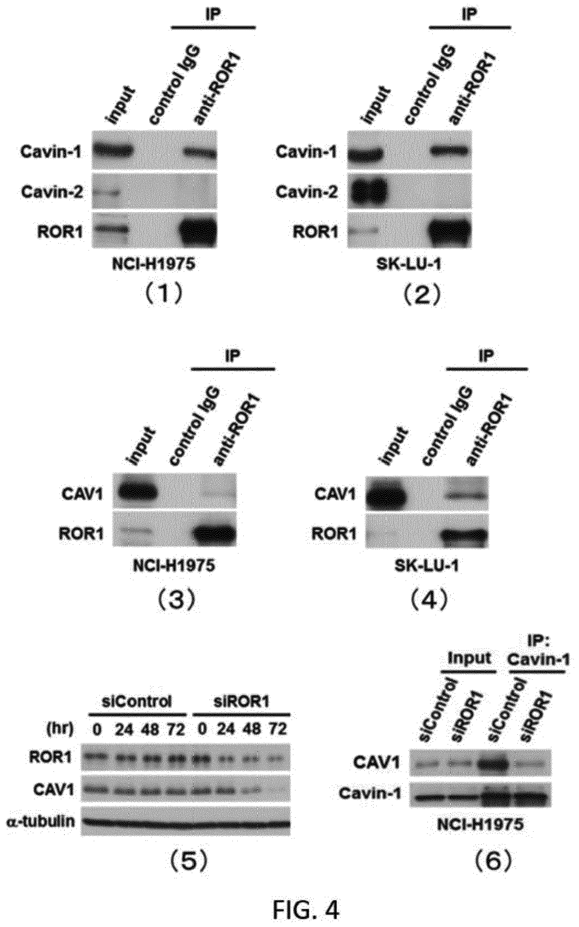

FIGS. 4(1) and (2) are photographs substituted for drawings and are photographs of Western blots showing that endogenous ROR1 and Cavin-1 bind, but ROR1 does not bind with Cavin-2 in NCI-H1975 and SK-LU-1, which are two different pulmonary adenocarcinoma cell lines. FIGS. 4(3) and (4) are photographs substituted for drawings and are photographs of Western blots showing that endogenous ROR1 and CAV1 also bind in NCI-H1975 and SK-LU-1, which are two different pulmonary adenocarcinoma cell lines. FIG. 4(5) is a photograph substituted for a drawing and is a photograph of a Western blot showing that in silencing of ROR1, expression of ROR1 is already lowered 24 hours after transfection of siROR1, while downregulation of CAV1 is seen after 48 hours. FIG. 4(6) is a photograph substituted for a drawing and is an IP-WB photograph showing that silencing of ROR1 decreases binding of endogenous CAV1 and Cavin-1.

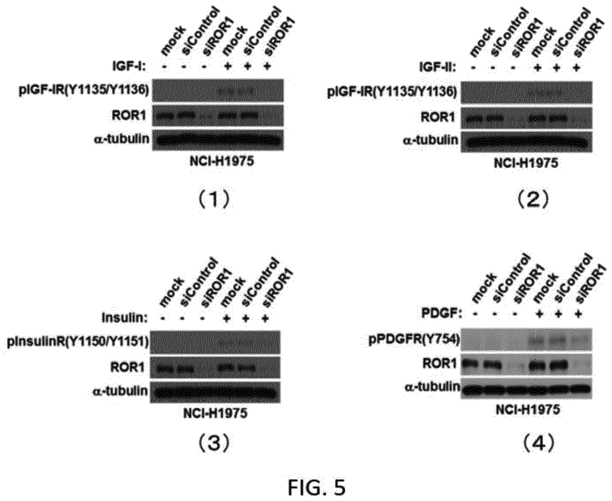

FIG. 5(1)-(4) are photographs substituted for drawings and are photographs of Western blots showing that silencing of ROR1 in NCI-H1975, a pulmonary adenocarcinoma cell line, lowers the phosphorylation reaction (activation) of various RTKs due to stimulation by various ligands.

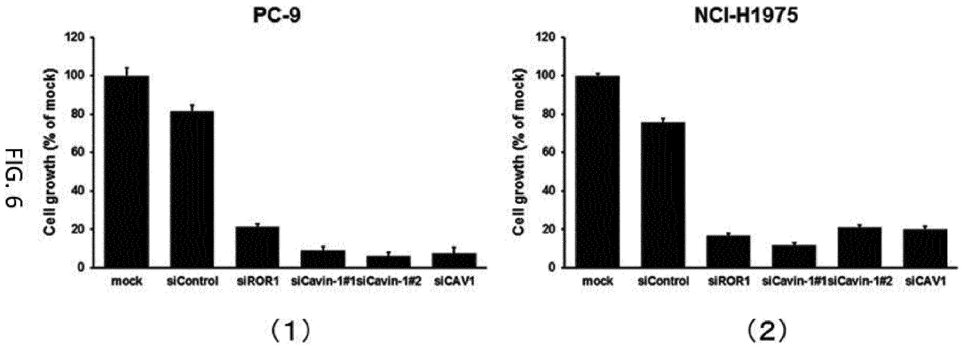

FIG. 6(1) and (2) are graphs showing by colorimetry that silencing of Cavin-1 inhibits cell proliferation in PC-9 and NCI-H1975, which are two different pulmonary adenocarcinoma cell lines. Furthermore, as reported previously, silencing of ROR1 inhibits cell proliferation (Patent Document 1 and Non-patent Document 5), and silencing of CAV1 is also confirmed to inhibit cell proliferation (Non-patent Document 7).

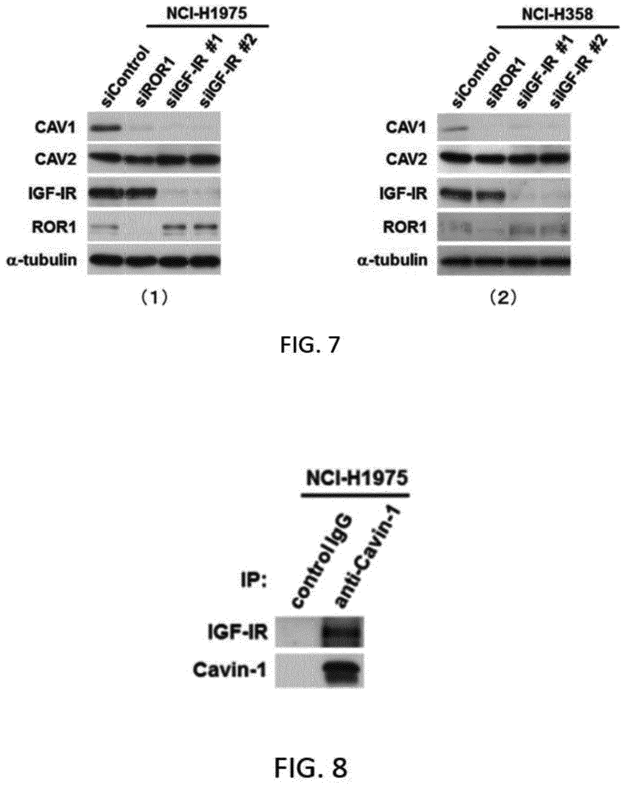

FIG. 7(1) and (2) are photographs substituted for drawings and are photographs of Western blots showing that downregulation of CAV1 was found due to silencing of IGF-IR in NCI-H1975 and NCI-H358, which are two different pulmonary adenocarcinoma cell lines.
FIG. 8 is a photograph substituted for a drawing and is a photograph of a Western blot showing that endogenous Cavin-1 and IGF-IR bind in NCI-H1975, which is a pulmonary adenocarcinoma cell line.

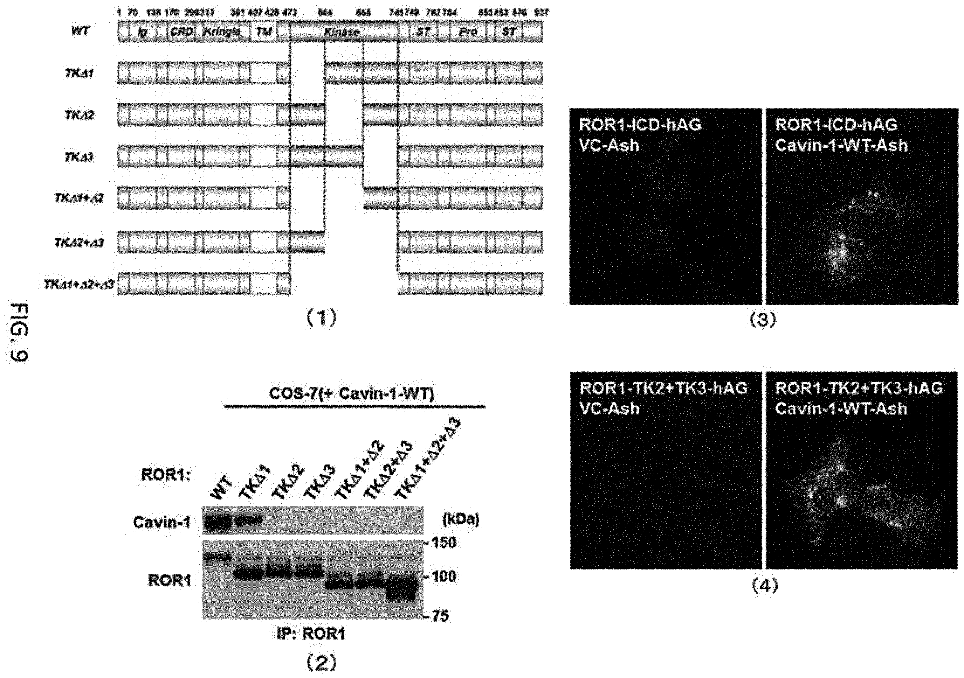

FIG. 9(1) shows a schematic diagram of each deletion mutant of the kinase domain of ROR1. FIG. 9(2) is a photograph substituted for a drawing and is a photograph of IP-WB analysis showing that Cavin-1 binds within the kinase domain of ROR1. FIG. 9(3) is a photograph substituted for a drawing and is a photograph showing the results of analysis by cell staining showing that the binding of the intracellular region of ROR1 and Cavin-1 can be detected intracellularly. FIG. 9(4) is a photograph substituted for a drawing and is a photograph showing the results of analysis by cell staining showing that binding of part of a region (region from amino acid 564 to amino acid 746) within the kinase domain of ROR1 and Cavin-1 can be detected intracellularly.

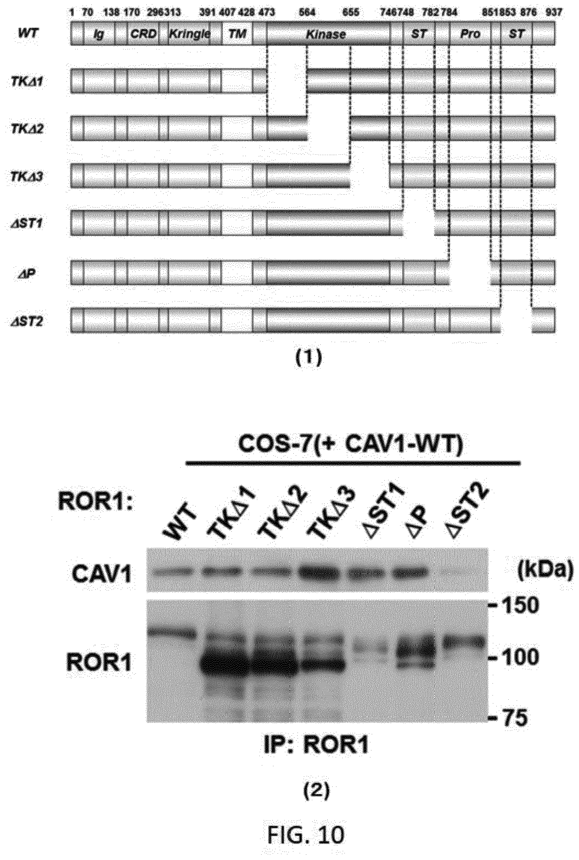

FIG. 10(1) is a schematic drawing of each deletion mutant of the ROR1 kinase domain, ST domain, and Pro domain. FIG. 10(2) is a photograph substituted for a drawing and is a photograph of IP-WB analysis showing that CAV1 binds in the serine-threonine domain located on the C-terminal side of the intracellular region of ROR1.

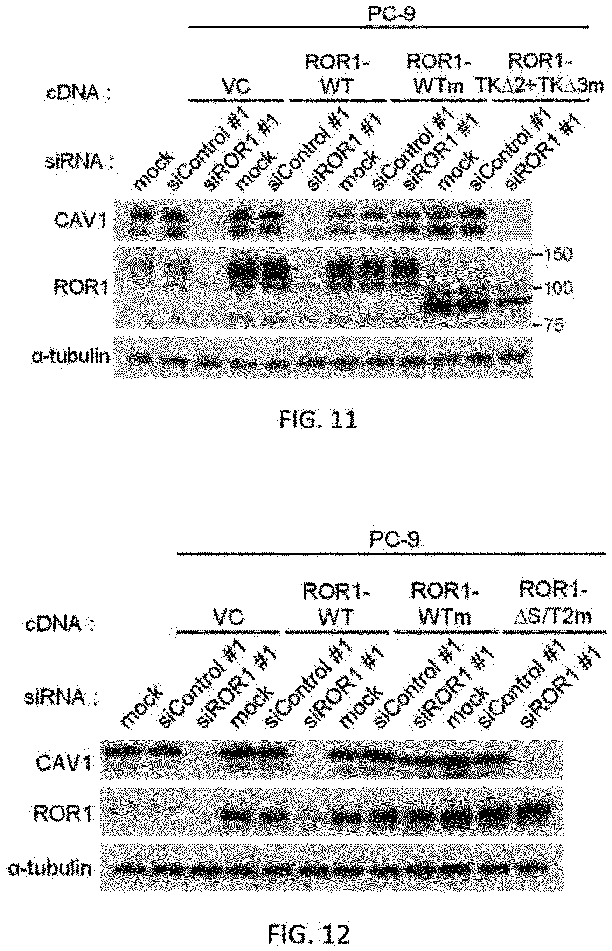

FIG. 11 is a photograph substituted for a drawing and is a photograph of a Western blot showing lowering of CAV1 expression when a mutant was prepared by adding a mutation cancelling the effect of siROR1 to a mutant from which the ROR1 binding region that binds to cavin-1 (TK2+TK3; amino acids 564 to 746) had been deleted, expressed within the cells, and subjected to siROR1 treatment, in PC-9 as a pulmonary adenocarcinoma cell line.
FIG. 12 is a photograph substituted for a drawing and is a photograph of a Western blot showing lowering of CAV1 expression when a mutant was prepared by adding a mutation cancelling the effect of siROR1 to a mutant from which the ROR1 binding region that binds to CAV1 (S/T2; amino acids 853 to 876) had been deleted, expressed within the cells, and subjected to siROR1 treatment, in PC-9 as a pulmonary adenocarcinoma cell line.

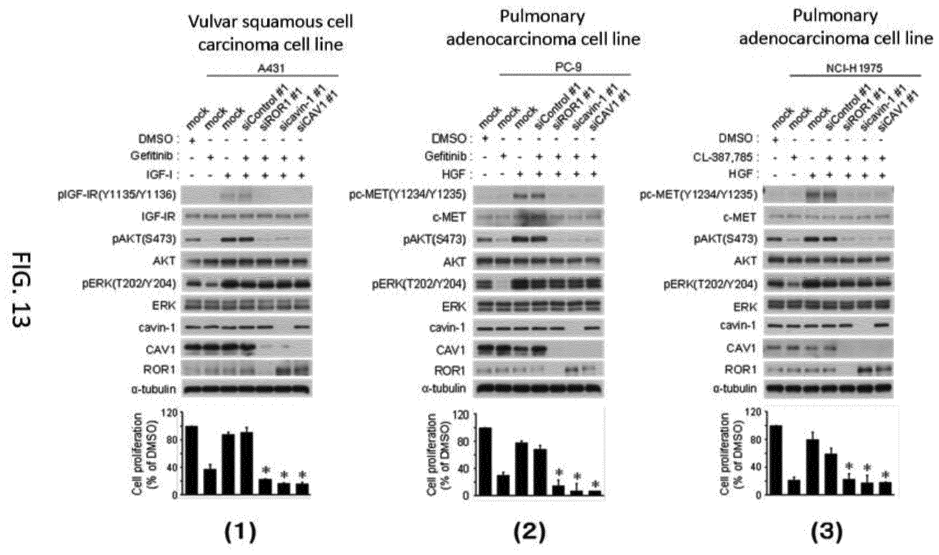

The lower part of FIG. 13(1) is a graph representing the results of MTT assay showing lowering of cell proliferation due to silencing of ROR1 in the presence of IGF-I and gefitinib using A431 as a vulvar squamous cell carcinoma cell line. The upper part of FIG. 13(1) is a photograph substituted for a drawing and is a photograph of a Western blot showing lowering of the phosphorylation of the AKT and IGF-IR involved in the survival signal. The lower part of FIG. 13(2) is a graph representing the results of MTT assay showing lowering of cell proliferation due to silencing of ROR1 in the presence of HGF and gefitinib using PC-9 as a pulmonary adenocarcinoma cell line. The upper part of FIG. 13(2) is a photograph substituted for a drawing and is a photograph of a Western blot showing lowering of the phosphorylation of the AKT and MET involved in the survival signal. The lower part of FIG. 13(3) is a graph representing the results of MTT assay showing lowering of cell proliferation due to silencing of ROR1 in the presence of HGF and CL-387, 785 using NCI-H1975 as a pulmonary adenocarcinoma cell line. The upper part of FIG. 13(3) is a photograph substituted for a drawing and is a photograph of a Western blot showing lowering of the phosphorylation of the AKT and MET involved in the survival signal.

### DESCRIPTION OF THE PREFERRED EMBODIMENTS

The method for screening a compound specifically suppressing the formation of caveolae of cancer cells, screening kit, vector and transformant to be used in the kit, and method for selecting a patient for whom a molecularly targeted drug is indicated of the present invention are explained in detail below.

First, the phrase "caveolae of cancer cells" in the present invention means dented structures found on the epithelial cells and endothelial cell membrane surface of cancer cells. RTKs that transmit signals intracellularly by binding with various growth factors such as EGF, PDGF, FGF, HGF, and insulin-like growth factor (IGF-IR) accumulate in the caveolae and function as signal transmission to cancer cells, uptake of lipids, and removal of bacteria that have invaded the body, and the like. The present invention is characterized by screening for a compound capable of suppressing the activity of various RTKs all at once as a result of suppressing the formation of the caveolae themselves rather than suppressing the activity of the individual RTKs accumulated in the caveolae.

The phrase "compound that specifically suppresses the formation of caveolae of cancer cells" in the present invention means a compound capable of specifically suppressing the formation of caveolae of cancer cells without affecting the formation of caveolae of normal cells.

CAV1 is known to contribute to the formation of caveolae (see Non-patent Document 6). Silencing (siCAV1) of CAV1 is also already known to be capable of suppressing the function of CAV1 and formation of caveolae (Non-patent Document 7). However, there is a risk that merely suppressing the function of CAV1 will also suppress the formation of caveolae of normal cells, but no factor that specifically suppresses the function of CAV1 of cancer cells alone was known.

It was newly discovered in the present invention that focusing on ROR1, which is a protein specific to cancer cells, makes it possible to specifically suppress the formation of caveolae of cancer cells alone without affecting the formation of caveolae of normal cells. Specifically, the binding of Cavin-1 and CAV1 necessary for the stabilization of CAV1 is suppressed by suppressing the binding of ROR1 and Cavin-1 or ROR1 and CAV1. As a result, the function of CAV1 that plays an important role in the formation of caveolae is suppressed. Thus, the formation of caveolae of cancer cells alone can be suppressed specifically since the formation of caveolae is also suppressed when the function of CAV1 is suppressed.

Furthermore, it was discovered for the first time in the present invention that the formation of caveolae of cancer cells alone can be suppressed specifically by focusing on ROR1, which is a protein specific to cancer cells. Therefore, with regard to the phrase "specific suppression of formation of caveolae of cancer cells" in the present invention, the target factor of the compound is not limited to ROR1 as long as the formation of caveolae of cancer cells can be suppressed in the end. For example, the phrase is not particularly restricted as long as the formation of caveolae of cancer cells can be specifically suppressed in the end, such as by suppressing the function of Cavin-1 or CAV1 by factors other than ROR1 such as IGF-IR, directly suppressing the function of Cavin-1 or CAV1, or the like.

Examples of the compound that serves as the target of the screening method of the present invention include natural compounds, organic compounds, inorganic compounds, proteins, antibodies, peptides, and other such single compounds, compound libraries, expression products of gene libraries, cell extracts, cell culture supernatants, fermented microbial products, marine organism extracts, plant extracts, and the like. In addition, "suppression" means not only complete suppression (that is, inhibition), but also includes partial suppression. For example, "suppression of the function of Cavin-1" includes both suppression of the activity and suppression of the expression of Cavin-1.

The genetic information, cDNA sequence, and amino acid sequence of human ROR1 are known. For example, the genetic information of GenBank Gene ID: 4919, the cDNA described in GenBank accession no. NM_005012, and the amino acid sequence information described in GenBank accession no. NP_005003 are available.

Caveolin (CAV) is known as a major protein for forming caveolae, and CAV1-3 are known. In the CAV family, CAV1 is known as a more important molecule as regards the formation of caveolae than CAV2 and CAV3 (see Non-patent Document 6). The genetic information, cDNA sequence, and amino acid sequence of human CAV1 are also known. For example, the genetic information of GenBank Gene ID: 857, cDNA described in GenBank accession no. NM_001753, and amino acid sequence information described in GenBank accession no. NP_001744 are available.

Cavin-1 is known to stabilize (suppress degradation by lysosomes) CAV1 by binding with the CAV1 protein (see Non-patent Document 6). The genetic information, cDNA sequence, and amino acid sequence of human Cavin-1 are known. For example, the genetic information of GenBank Gene ID: 284119, cDNA described in GenBank accession no. NM_012232, and amino acid sequence information described in GenBank accession no. NP_036364 are available.

Potential applications to cancer cells are believed to be wide, as long as the cancer cells form caveolae and accumulate RTKs in the caveolae. Examples include, but are not limited to, cells of cancers such as lung cancer, pulmonary adenocarcinoma, pancreatic cancer, malignant mesothelioma, breast cancer, colon cancer, oral cavity cancers, esophageal cancer, and the like. Examples of species from which the cancer cells are derived include, but are not limited to, humans, monkeys, mice, rats, guinea pigs, pigs, cows, sheep, goats, and the like.

The screening method of the present invention is not particularly restricted as long as it can bring a test compound into contact with a system capable of detecting directly or indirectly whether the formation of caveolae of cancer cells can be suppressed, and detect changes in function before and after contact.

For example, a test compound is brought into contact with a system that expresses Cavin-1, a system that expresses CAV1, a system that expresses CAV1 and Cavin-1, a system that expresses ROR1 and Cavin-1, a system that expresses ROR1 and CAV1, a system that expresses IGF-IR and Cavin-1, a system that expresses IGF-IR and CAV1, or the like. Next, the existence of lowering of the expression of Cavin-1, lowering of the expression of CAV1, suppression of the binding of CAV1 and Cavin-1, suppression of the binding of ROR1 and Cavin-1, suppression of the binding of ROR1 and CAV1, suppression of the binding of IGF-IR and Cavin-1, or suppression of the binding of IGF-IR and CAV1 may be detected using a known detection method. Furthermore, the screened compound does not affect normal cells when a test compound is brought into contact with a system that expresses ROR1 since ROR1 is a protein specific to cancer cells. On the other hand, lowering of the expression of Cavin-1, lowering of the expression of CAV1, suppression of the binding of CAV1 and Cavin-1, suppression of the binding of IGF-IR and Cavin-1, and suppression of the binding of IGF-IR and CAV1 in normal cells are also conceivable when a test compound is brought into contact with a system that does not express ROR1, for example, a system that expresses only Cavin-1, a system that expresses only CAV1, a system that expresses Cavin-1 and CAV1, a system that expresses IGF-IR and Cavin-1, or a system that expresses IGF-IR and CAV1. One should therefore administer the screened compound to cancer cells and normal cells and confirm whether it is specific to cancer cells when screening has been conducted using a system that does not express ROR1.

The detection method can be, for example, ELISA (enzyme immunoassay) or immunoprecipitation-Western blotting (IP-WB). The test compound is judged to specifically suppress caveolae formation of cancer cells if the expression of Cavin-1 is lowered, the expression level of CAV1 is lowered, or the binding level of CAV1 and Cavin-1 is lowered, the binding level of ROR1 and Cavin-1 is lowered, the binding level of ROR1 and CAV1 is lowered, the binding level of IGF-IR and Cavin-1 is lowered, or the binding level of IGF-IR and CAV1 is lowered in comparison to that detected in the absence of the test compound.

In addition to the detection methods described above, a compound that specifically suppresses caveolae formation of cancer cells can also be screened as the fluorescent brightness of a protein using the screening kit of the present invention. The binding site of ROR1 that binds with Cavin-1 was newly identified in the present invention to be an amino acid region 564 to 746 within the kinase domain within the intracellular region of ROR1 (ROR1-ICD: amino acid sequence 428 to 937). Since Cavin-1 binds to the intracellular region of ROR1, cells that express at least the amino acid region of the amino acid sequence 564 to 746 within the amino acid sequence of the intracellular region of ROR1 and Cavin-1 are prepared, and a system capable of detecting intracellular binding of the proteins expressed as fluorescent brightness is constructed for the screening kit of the present invention. The binding site of ROR1 that binds with CAV1 was also newly identified in the present invention to be an amino acid region 853 to 876 within the kinase domain within the intracellular region of ROR1. In the same way as described above, cells that express at least the amino acid region of the amino acid sequence 853 to 876 and CAV1 are prepared, and a system capable of detecting the intracellular binding of the proteins expressed as fluorescent brightness can be constructed.

Cells that express both the amino acid region 564 to 746 of ROR1 and Cavin-1 can be prepared by the following procedure.
(1) A recombinant vector into which a nucleic acid that encodes an amino acid sequence including the amino acid region 564 to 746 within the amino acid sequence 428 to 937 of the intracellular region of ROR1 has been inserted is prepared.
(2) A recombinant vector into which a nucleic acid that encodes an amino acid sequence of human Cavin-1 has been inserted is prepared.
(3) Transformants are prepared by introducing the vectors of (1) and (2) into cells.
   The recombinant vectors can be prepared and the recombinant vectors can be introduced into cells by known methods. HeLa cells, HEK293 cells, COS-7 cells, CHO cells, and the like can be used as cells.
(4) A cell line capable of constitutive expression is prepared as a transformant into which the recombinant vectors of (1) and (2) have been introduced using these cells.

Next, a screening kit of the present invention can be prepared, for example, by using the above transformant in a known kit capable of detecting the intracellular binding of proteins expressed as fluorescent brightness, such as image-based protein-protein interaction analysis (Fluoppi), Fluo-chase, or the like. The test compound can be selected easily because binding of the binding site of the ROR1 expressed to Cavin-1 with Cavin-1 can be detected as fluorescent brightness (foci: dot structures), and it is not detected as fluorescent brightness when the test compound is capable of suppressing the binding of the ROR1 binding site and Cavin-1.

Cells that express both the amino acid region 853 to 876 of ROR1 and CAV1 may contain at least the amino acid region 853 to 876 instead of the amino acid region 564 to 746 of step (1) above, and a nucleic acid that encodes an amino acid sequence of human CAV1 may be used instead of the nucleic acid that encodes an amino acid sequence of human Cavin-1 of step (2) above.

Furthermore, the detection system is generally said to be unstable when the full length of the nucleic acid encoding the protein is incorporated into the vector when membrane proteins (for example, ROR1 protein) and proteins that are normally dispersed or localized in dots in cells (for example, Cavin-1 protein) are incorporated in image-based protein-protein interaction analysis. Since the binding regions of ROR1 with Cavin-1 and CAV1 were determined to be part of the intracellular region, the amino acid sequence 1 to 427 portion, which is the extracellular region portion of ROR1, is not incorporated, but the nucleic acid portion that encodes the amino acid sequence of the extracellular region portion may be incorporated into the vector as long as it is within the range that stabilizes the detection system in the present invention. In addition, the vector may be constructed to express the binding site of ROR1 to Cavin-1 or the binding site of ROR1 to CAV1 intracellularly. For example, the combination of regions expressed and proteins may be decided as is appropriate in accordance with the screening goal, such as incorporating into the vector so that both the binding site of ROR1 to Cavin-1 and the binding site of ROR1 to CAV1 are expressed intracellularly. Furthermore, when both the binding site of ROR1 to Cavin-1 and the binding site of ROR1 to CAV1 are expressed intracellularly, a transformant may be prepared so that both the amino acid sequence of human Cavin-1 and the amino acid sequence of human CAV1 are expressed within the transformant. Methods utilizing immunoprecipitation, a yeast two-hybrid system, FRET (fluorescence resonance energy transfer), and surface plasmon resonance can also be used as other detection methods.

In addition, for the method for selecting a patient for whom a molecularly targeted drug of the present invention is indicated, a patient for whom a compound screened by the above method for screening a compound that specifically suppresses formation of caveolae of cancer cells is indicated can be selected by measuring the expression of at least one selected from ROR1 and Cavin-1, ROR1 and CAV1, IGF-IR and Cavin-1, IGF-IR and CAV1, Cavin-1 and CAV1, CAV1, or Cavin-1 in a sample.

A known method such as ELISA, IP-WB, or the like may be used for the expression of at least one selected from ROR1 and Cavin-1, ROR1 and CAV1, IGF-IR and Cavin-1, IGF-IR and CAV1, Cavin-1 and CAV1, CAV1, or Cavin-1 in a sample.

The present invention is explained concretely below through examples. These examples, however, are provided merely as a reference to concrete embodiments to explain the present invention. These examples are intended to explain specific concrete embodiments of the present invention, but do not represent any limitation or restriction of the scope of the invention disclosed in this application.

### EXAMPLES

### <Materials and method>

### (1) Cell lines and reagents

NCI-H1975 (ATCC accession no.: CRL-5908), NCI-H358 (ATCC accession no.: CRL-5087), NCI-H441 (ATCC accession no.: HTB-174), and COS-7 (ATCC accession no.: CRL-1651), which are human pulmonary adenocarcinoma cell lines, A431 (ATCC accession no.: CRL-1555) which is a human vulvar squamous cell carcinoma cell line, and HeLa (ATCC accession no.: CCL-2) were purchased from the American Type Culture Collection. The PC-9 cell line (RCB4455), which is a human pulmonary adenocarcinoma cell line, was obtained from the Riken Cell Bank. The SK-LU-1 cell line which is a human pulmonary adenocarcinoma cell line was provided by Lloyd J. Old (Memorial Sloan Kettering Cancer Center). These cell lines were maintained by RPMI 1640 (manufactured by Invitrogen) with 10% fetal bovine serum (FBS) added.

Recombinant insulin, recombinant IGF-I, recombinant IGF-II, recombinant HGF, and recombinant PDGF were obtained from PeproTech Inc. Gefitinib, which is EGFR-TKI, was obtained from Biaffin GmbH & Co. KG, and CL-387, 785 was obtained from Calbiochem Corp.

A phospho-RTK array kit was obtained from R&D System Co. A Fluoppi: Ash-hAG (Ash-MNL/MCL+hAG_MNL/MCL) kit was obtained from MBL.

The following antibodies were purchased from the following companies.
- anti-ROR1, anti-IGF-IR, anti-phospho-insulinR (Y1150/Y1151), anti-phospho-IGF-IR (Y1135/Y1136), anti-phospho-PDGF (Y754), anti-phospho-c-Met (Y1234/Y1235), anti-pAKT (S473), anti-AKT, anti-phospho-ERK (T202/Y204), anti-ERK, and anti-Met were purchased from Cell Signaling Technology Co.
- anti-Cavin-1 and anti-Cavin-2 were purchased from Abcam Co.
- anti-α-tubulin was purchased from Sigma Inc.
- anti-ROR1 (for immunoprecipitation) was purchased from R&D System Co.
- anti-CAV1 and anti-CAV2 were purchased from Bioscience Inc.
- anti-mouse IgG and anti-rabbit IgG were purchased from Signaling Technology Co.

### (2) Construct

Human ROR1 full-length cDNA (manufactured by OriGene Technologies Inc.) was inserted into a pCMV-puro vector. The open reading frame (ORF) sequence of the construct (pCMVpuro-ROR1) obtained was confirmed exactly. Human Cavin-1 full-length cDNA (manufactured by OriGene Technologies Inc.) was inserted into a pCMV-puro vector. The open reading frame (ORF) sequence of the construct (pCMVpuro-Cavin-1) obtained was confirmed exactly. Human CAV1 full-length cDNA (manufactured by OriGene Technologies Inc.) was inserted into a pCMV-puro vector. The open reading frame (ORF) sequence of the construct (pCMVpuro-CAV1) obtained was confirmed exactly. pCMVpuro-ROR1-ICD (from amino acid 428 to amino acid 937) and pCMVpuro-ROR1-TK2+TK3 (region from amino acid 564 to amino acid 746) were prepared by a molecular biological approach using restriction enzymes.

In vitro mutagenesis was conducted using KOD-plus-DNA polymerase (Toyobo Co., Ltd.), and pCMVpuro-ROR1-TKΔ473-564, pCMVpuro-ROR1-TKΔ564-655, pCMVpuro-ROR1-TKΔ655-746, pCMVpuro-ROR1-TKΔ473-655, pCMVpuro-ROR1-TKΔ564-746, pCMVpuro-ROR1-TKΔ473-746, pCMVpuro-ROR1-ST1Δ748-782, pCMVpuro-ROR1-PΔ784-851, and pCMVpuro-ROR1-ST2Δ853 to 876 were prepared. The primers used in preparation are shown below.
Δ746 forward primer :5'-CGCTCCTGGGAGGGACTCTCAAGTC-3'(SEQ ID No: 1),
Δ564 forward primer:5'-ATCATGAGATCCCCACACTCTGATG-3'(SEQ ID No: 2),
Δ655 forward primer:5'-ATTCGCTGGATGCCCCCTGAAGCCA-3'(SEQ ID No: 3),
Δ748 forward primer:5'-AACCCCAGATATCCTAATTACATGT-3'(SEQ ID No: 4)
Δ851 forward primer:5'-AAGAGTCGGTCCCCAAGCAGTGCCA-3'(SEQ ID No: 5),
Δ853 forward primer:5'-AATCAGGAAGCAAATATTCCTTTAC-3'(SEQ ID No: 6),
Δ473 reverse primer:5'-AGCAGAAAGAGGTAGCTCTTTAGCC-3'(SEQ ID No: 7),
Δ564 reverse primer:5'-GAGGAACTCATGGAGATCCCCCTGA-3'(SEQ ID No: 8),
Δ655 reverse primer:5'-GGGCAGCAAGGACTTACTCTGGACC-3'(SEQ ID No: 9),
Δ782 reverse primer:5'-CCGAAGCCGGACGTGAATATCTTTA-3'(SEQ ID No: 10),
Δ782 reverse primer:5'-GTTACTGAGATTACTCACTGGGCTG-3'(SEQ ID No: 11),
Δ876 reverse primer:5'-CTTGGGAGGTGGGCAGTGCTGAATC-3'(SEQ ID No: 12)

In vitro mutagenesis was conducted using KOD-plus-DNA polymerase (Toyobo Co., Ltd.), and pCMVpuro-ROR1-KD (kinase activity deficient type; K506A) was prepared.

### (3) Western blotting analysis and IP-WB analysis

Western blotting analysis and IP-WB analysis were conducted by the standard methods using an Immobilon-P filter (manufactured by Millipore Corp.) and an enhanced chemiluminescence system (manufactured by GE Healthcare Inc.). pCMVpuro-Cavin-1-WT was coexpressed with various ROR1 expression constructs such as the wild type (WT), TKΔ473-564 (TKΔ1), TK564-655 (TKΔ2), TKΔ655-746 (TKΔ3), TKΔ473-655 (TKΔ1+Δ2), TKΔ564-746 (TKΔ2+Δ3), and TKΔ473-746 (TKΔ1+Δ2+Δ3) to identify the binding site of ROR1 and Cavin-1. pCMVpuro-CAV1-WT was coexpressed with ROR1 expression constructs such as the wild type (WT), TKΔ473-564 (TKΔ1), TKΔ564-655 (TKΔ2), TKΔ655-746 (TKΔ3), ST1Δ748-782 (ΔST1), PΔ784-851 (ΔP), and ST2Δ853 to 876 (ΔST2) to identify the binding site of ROR1 and CAV1. For these, the cells were recovered 24 hours after transfection by Fugene6 (Promega) using COS-7 cells, and IP-WB analysis was conducted.

To study the RTK phosphorylation reaction (activation) by stimulation by each ligand due to silencing of ROR1, the culture broth was removed 42 hours after transfection of siControl#1 (sometimes referred to as "siControl" hereinafter in the description and drawings) and siROR1#1 (sometimes referred to as "siROR1" hereinafter in the description and drawings) using NCI-H1975 cells. After washing with PBS, the culture broth was replaced by a serum (FBS: fetal bovine serum)-free broth, and cultured for another 24 hours. The cells were then recovered after treating for 30 minutes by 20ng/mL of Insulin, IGF-I, IGF-II, or PDGF, and Western blotting analysis was conducted.

In addition, IP-WB analysis was conducted after suspending the cells in lysis buffer containing octyl glucoside (10 mM Tris-HCl (pH 8.0), 0.15 M NaCl, 5 mM EDTA, 1% Triton X-100, 60 mM octyl glucoside) using NCI-H1975, NCI-H358, and SK-LU-1 cell lines, which are pulmonary adenocarcinoma cell lines, to confirm the binding of ROR1 and Cavin-1, ROR1 and CAV1, or Cavin-1 and IGF-IR.

### (4) Immunofluorescent staining

Cells obtained by transfection into HeLa cells by Fluoppi were examined by a fluorescence microscope (manufactured by Leica Inc.) after removing the culture broth after 24 hours, washing with PBS, fixing by 3.7% formalin solution, and preparing prepared specimens.

### (5) phospho-RTK array

The cells of the NCI-H1975 and PC-9 cell lines were recovered after removing the culture broth 72 hours after transfections of siControl or siROR1 and washing with PBS, and analyzed by the R&D Co. protocol after suspension in NP-40 lysis buffer (1% NP-40, 20 mM Tris-HCl (pH 8.0), 137 mM NaCl, 10% glycerol, 2mM EDTA, 1 mM sodium orthovanadate, protein inhibitor tablet (Roche Inc.)).

### (6) RNA interference (RNAi) against human ROR1, human IGF-IR, human Cavin-1, and human CAV1

The following RNA oligomers were obtained from Qiagen Inc. and Sigma-Aldrich Co. to conduct RNAi.
- 5' -CAGCAAUGGAUGGAAUUUCAA-3' :siROR1#1 (SEQ ID No: 13),
- 5' -CCCAGUGAGUAAUCUCAGU-3' :siROR1#2 (SEQ ID No: 14),
- 5' -CCCAGAAGCUGCGAACUGU-3' :siROR1#3 (SEQ ID No: 15),
- 5' -GGAAUUGCAUGGUAGCCGAUU-3' :siIGF-IR#1 (SEQ ID No: 16),
- 5' -CUGACUACAGGGAUCUCAUUU-3' :siIGF-IR#2 (SEQ ID No: 17),
- 5'-CUCCAAGACCGCGGUCUACAA-3' :siCavin-1#1 (SEQ ID No: 18),
- 5'-CCCGCCGAGCGGCGCGAGAAA-3' :siCavin-1#2 (SEQ ID No: 19),
- 5'-AGACGAGCUGAGCGAGAAGCA-3' :siCAV1#1 (SEQ ID No: 20)

All Stars negative control siRNA (siControl#1) was also obtained from Qiagen Inc. as a control. siControl#2 was also obtained from Sigma-Aldrich Co.

Transfection of the siRNA (20 nM each) was conducted in accordance with the manufacturer's instruction manual using lipofectamine RNAiMAX (manufactured by Invitrogen). Cells were recovered 72 hours after transfection for Western blotting analysis. Cells were also recovered 120 hours after transfection for analysis by colorimetry to measure cell proliferation.

### (7) Study of the importance of the kinase activity of ROR1 in CAV1 expression regulation

In vitro mutagenesis was conducted using KOD-plus-DNA polymerase (Toyobo), and pCMVpuro-ROR1-WTm (siROR1) and pCMVpuro-ROR1-KDm (siROR1) including silent mutations for siROR1 were prepared. Following primer of not involving amino acid substitution was used for production.

### •5'-CAACAGTGGACAGAGTTCCAG-3' : (SEQ ID NO: 21)

Wild type ROR1 (ROR1-WT), wild type ROR1 with an added silent mutation for siROR1 (ROR1-WTm), or kinase activity-deficient ROR1 with a silent mutation for siROR1 added (ROR1-KDm) were transfected into NCI-H1975 cells. Selection by puromycin (1.5 µg/mL) was performed after 24 hours, and the single clones were collected, and stable clones that expressed the respective ROR1 (constitutive ROR1-expressing cell lines) were prepared. These stable clones were then treated by siControl or siROR1, and the cells were recovered after three days and Western blotting analysis was conducted.

### (8) Study of the importance of the cavin-1 binding region of ROR1 in CAV1 expression regulation

In vitro mutagenesis was conducted using KOD-plus-DNA polymerase (Toyobo), and pCMVpuro-ROR1-WTm (siROR1) and pCMVpuro-ROR1-TKΔ2+TKΔ3m (siROR1) including silent mutations for siROR1 were prepared. Following primer of not involving amino acid substitution was used for production.

### •5'-CAACAGTGGACAGAGTTCCAG-3': (SEQ ID NO: 21)

Wild type ROR1 (ROR1-WT), wild type ROR1 with an added silent mutation for siROR1 (ROR1-WTm), or cavin-1 binding region-deficient ROR1 with an added silent mutation for siROR1 (ROR1-TKΔ2+TKΔ3m) were transfected into PC-9 cells. Selection by puromycin (1.0 µg/mL) was performed after 24 hours, and the cells were recovered as bulk clones and reseeded in 6-well plates. The cells were then treated by siControl or siROR1, recovered after three days, and Western blotting analysis was conducted.

### (9) Study of the importance of the CAV1 binding region of ROR1 in CAV1 expression regulation

In vitro mutagenesis was conducted using KOD-plus-DNA polymerase (Toyobo), and pCMVpuro-ROR1-WTm (siROR1) and pCMVpuro-ROR1-ΔS/T2m (siROR1) including silent mutations for siROR1 were prepared. Following primer of not involving amino acid substitution was used for production.

### •5'-CAACAGTGGACAGAGTTCCAG-3' : (SEQ ID NO: 21)

Wild type ROR1 (ROR1-WT), wild type ROR1 with an added silent mutation for siROR1 (ROR1-WTm), or CAV1 binding region-deficient ROR1 with an added silent mutation for siROR1 (ROR1--ΔS/T2m) were transfected into PC-9 cells. Selection by puromycin (1.0 µg/mL) was performed after 24 hours, and the cells were recovered as bulk clones and reseeded in 6-well plates. The cells were then treated by siControl or siROR1, recovered after three days, and Western blotting analysis was conducted.

### (10) Study of the influence and effects on caveolae formation in ROR1-silenced cells by electron microscope analysis using rapid freezing/freeze-fracture replica labeling

20 µm thick gold foil was placed in a culture dish, and NCI-H1975 cells, which are a pulmonary adenocarcinoma cell line, were cultured on top. Transfection of siControl or siROR1 was conducted. Rapid freezing was conducted using a high-pressure freezing machine (Leica Inc.) 72 hours after transfection. Freeze-fracture replicas were prepared using a BAF400 apparatus (Baltec Co.), and labeled by mouse anti-CAV2 antibody (BD Transduction Co.) after treatment by SDS. A second antibody reaction was then conducted using 10 nm gold colloid-conjugated goat anti-mouse IgG antibody (British Biocell International Co.), and the cells were examined by a JEM-1011 electron microscope. For caveolae distribution density measurement, randomly selected regions under the electron microscope (18 random cell membrane regions of 17-50 µm) were measured, and the cell membrane region of each electron micrograph was measured using ImageJ.

### (11) Detection of binding of ROR1 and Cavin-1 using image-based protein-protein interaction analysis

The image-based protein-protein interaction analysis system construction and analytical method were in accordance with the protocol of the provider MBL. The following constructs were prepared by a molecular biological approach using restriction enzymes in accordance with the MBL protocol for pCMVpuro-ROR1-ICD (from amino acid 428 to amino acid 937), pCMVpuro-ROR1-TK2+TK3 (region from amino acid 564 to amino acid 746), and pCMVpuro-Cavin-1-WT (full length); phAG-MNL-ROR1-ICD, phAG-MNL-ROR1-TK2+TK3, phAG-MNL-Cavin-1-WT, phAG-MCL-ROR1-ICD, phAG-MCL-ROR1-TK2+TK3, phAG-MCL-Cavin-1-WT, pAsh-MNL-ROR1-ICD, pAsh-MNL-ROR1-TK2+TK3, pAsh-MNL-Cavin-1-WT, pAsh-MCL-ROR1-ICD, pAsh-MCL-ROR1-TK2+TK3, pAsh-MCL-Cavin-1-WT. The sequence was confirmed exactly by sequencing to confirm that each construct obtained was properly incorporated. Transfection (Fugene6 (Promega))by eight suitable combinations was conducted thereafter using HeLa cells. A plasmid that incorporated ROR1-ICD or ROR1-TK2+TK3 and a plasmid that did not incorporated Cavin-1-WT were also transfected simultaneously (four combinations, negative control). The cells were fixed by 3.7% formalin solution 24 hours after transfection and examined.

### (12) Study of the effects of silencing of ROR1, Cavin-1, and CAV1 by RNAi interference on cell proliferation for pulmonary adenocarcinoma cell lines

siControl or siROR1, siCavin-1, or siCAV1 was transfected against the PC-9 or NCI-H1975 cell line to investigate the effects of silencing of ROR1, Cavin-1, and CAV1 on cell proliferation. A reaction was conducted using a cell counting kit-8 (Dojindo) 120 hours after transfection, the absorbance was measured by a plate reader, and colorimetry was conducted. Furthermore, this experiment was conducted three separate times, and the average values were graphed.

### (13) Study of the effects of silencing ROR1, Cavin-1, and CAV1 on cell proliferation and influence on the survival signal in an EGFR-TKI-resistant cell line under ligand stimulation

The effects of silencing of ROR1, Cavin-1, and CAV1 on cell proliferation and the influence on the survival signal were investigated in a cell line that had acquired EGFR-TKI resistance in the presence of various clinically problematic ligands. After creating a state in which various ligands (50 ng/mL) and EGFR-TKI (gefitinib 1 µM, CL-387, 785 0.5 µM) were present (A431: gefitinib+IGF-I, PC-9: gefitinib+HGF, H1975: CL-387785+HGF), siControl or siROR1, siCavin-1, or siCAV1 were transfected (20 µM) against A431, which is vulvar squamous cell carcinoma cells, and PC-9 and NCI-H1975, which are pulmonary adenocarcinoma cells. In Western blotting, the cells were spread on a plate, then subjected to each siRNA treatment the following day. After three days, each ligand and EGFR-TKI were reacted for six hours, and the cells were recovered. In MTT assay, the cells were spread on a plate, then subjected to each siRNA treatment the following day. Each ligand and EGFR-TKI were reacted the following day, a reaction was conducted four days later using a cell counting kit-8 (Dojindo), the absorbance was measured by a plate reader, and colorimetry was conducted. Furthermore, this experiment was conducted three separate times, and the average values were graphed.

### [Example 1]

### <Verification relating to the influence of ROR1 silencing on associated RTK activation in pulmonary adenocarcinoma cell lines>

The study was conducted by phospho-RTK array analysis using NCI-H1975 and PC-9 cell lines, which are pulmonary adenocarcinoma cell lines, by the procedure shown in (5) above. FIG. 1(1) is a photograph showing the analysis results of NCI-H1975, and FIG. 1(2) shows the results of PC-9. As shown in FIG. 1(1), mainly lowering of the phosphorylation reaction of the ERBB2, ERBB3, PDGFR, c-KIT, ALK, insulinR, and IGF-IR RTKs was found in NCI-H1975. As shown in FIG. 1(2), mainly lowering of the phosphorylation reaction of the ERBB2, ERBB3, IGF-IR, c-MET, MSPR, and EphR2 RTKs was found in the PC-9 cell line. It was understood from the above results that silencing of ROR1 can lower the phosphorylation reaction (activation) of many different RTKs. Examples of especially remarkable lowering of RTK phosphorylation seen in common in the two cell lines are ERBB2, ERBB3, and IGF-IR. The above results show that the expression of ROR1 is involved in the phosphorylation reaction of a wide range of many RTKs and thus in maintenance of the activation of RTKs in pulmonary adenocarcinoma cell lines, and the possibility of a common RTK activation mechanism via ROR1. Furthermore, the RTKs on which an influence was seen are responsible for important physiological functions such as survival signal in pulmonary adenocarcinoma cells. Therefore, since silencing of ROR1 comprehensively suppresses these RTKs, it became clear that ROR1 has the potential to serve as a completely unprecedented innovative molecular target that can arrest all at once the activation of a variety of RTKs responsible for important roles for pulmonary adenocarcinoma.

### [Example 2]

### <Study relating to downregulation of CAV1 due to silencing of ROR1 and influence on caveolae formation>

The downregulation of CAV1 due to silencing of ROR1 in various adenocarcinoma cell lines and the influence on caveolae formation were analyzed by Western blotting by the procedure shown in (6) above. FIG. 2(1) shows the results obtained when using NCI-H1975 as the pulmonary adenocarcinoma cell line. Silencing of ROR1 using the different sequences siROR1#1-3 was found to downregulate the CAV1 that is essential to the formation of caveolae in all cases. This result clarified that downregulation of CAV1 is due to silencing of ROR1 rather than a specific phenomenon based on a specific sequence of siROR1. FIG. 2(2) shows the results obtained when using NCI-H441, NCI-H358, and PC-9 as pulmonary adenocarcinoma cell lines. As in FIG. 2(1), downregulation of CAV1 was found in each cell line when the expression of ROR1 was suppressed using siControl and siROR1. These results indicate the possibility that the regulation of CAV1 expression by ROR1 may have a common regulatory mechanism in many pulmonary adenocarcinoma cell lines.

Moreover, since downregulation of CAV1 was found in association with silencing of ROR1 in pulmonary adenocarcinoma cell lines, electron microscopic analysis using freeze/fracture replica labeling was conducted by the procedure of (10) above using NCI-H1975 as the pulmonary adenocarcinoma cell line or using siControl and siROR1 to study whether there is an influence on caveolae formation. FIG. 2(3) is a photograph showing the results of analysis by electron microscopic analysis. As shown in FIG. 2(3), it was clarified that silencing of ROR1 inhibited formation of caveolae on the cell membrane and that the number of normal caveolae decreased. Furthermore, labeling of caveolae was conducted by CAV2, and gold colloid-labeled CAV2 accumulated in structures that appeared to be traces of caveolae (structures with a shallow indentation) in cell lines in which the expression of ROR1 was suppressed. FIG. 2(4) is a graph showing the results obtained by measuring the number of deeply indented structures, that is, normal caveolae, in random regions on the cell membrane in NCI-H1975 that had been treated by siControl and siROR1 (mean±standard deviation (n=18, where n is the random region on the cell membrane of 17-50 µm²)). As shown by the graph in FIG. 2(4), it was clarified that the number of normal caveolae decreases significantly in cell lines treated by siROR1. It was judged based on the above results that the expression of ROR1 is indispensable to the formation of normal caveolae in pulmonary adenocarcinoma cell lines.

### [Example 3]

### <Study of the necessity of the kinase activity of ROR1 in expression regulation of CAV1 by ROR1>

The present inventors reported that ROR1, which is an RTK, regulates the PI3K-AKT axis related to the survival signal of cancer cells in a kinase activity dependent or independent manner in pulmonary adenocarcinoma cell lines (see the above Patent Document 2), and studied whether the kinase activity of ROR1 is necessary for expression regulation of CAV1 by ROR1. An experiment was conducted by the procedure of (7) above using a pulmonary adenocarcinoma cell line NCI-H1975 that constitutively expresses ROR1-WT (wild type ROR1), ROR1-WTm (wild type ROR1 with a silent mutation added against siROR1), and ROR1-KDm (kinase activity-deficient ROR1 with a silent mutation added against siROR1). FIG. 3 is a photograph showing the results obtained by analysis by Western blotting. As shown in FIG. 3, downregulation of CAV1 previously confirmed by siROR1 treatment was found in cell lines that expressed ROR1-WT.

In contrast, downregulation of CAV1 was not seen due to siROR1 treatment in cell lines that expressed ROR1-WTm. This shows that siROR1 works normally within the cell and triggers downregulation of CAV1 as a function of ROR1 within the cell. Moreover, downregulation of CAV1 was not seen due to siROR1 treatment either in cell lines that expressed ROR1-KDm. It is understood from these results that the kinase activity of ROR1 is not necessary for downregulation of CAV1 by siROR1. In other words, it was judged that the kinase activity of ROR1 is not important in expression regulation of CAV1 via ROR1. This shows that identification of ROR1 function inhibitors focusing on the kinase activity-independent function of ROR1 and screening methods for identifying these inhibitors are necessary.

### [Example 4]

### <Study of the binding of ROR1 and the caveolae constituent factors Cavin-1 and CAV1 and the importance of ROR1 in the binding of CAV1 and Cavin-1 in pulmonary adenocarcinoma cell lines>

The expression of CAV1 which is necessary for the formation of caveolae is known to be stabilized by binding with Cavin-1 (see Non-patent Documents 8-10). In other words, Cavin-1 is understood to be a very important protein in the formation of caveolae. It remains unclear, however, through what kind of regulation Cavin-1 stabilizes the expression of CAV1 in the formation of caveolae. Moreover, the detailed functions of Cavin-1 are not well understood. The present inventors therefore focused on Cavin-1 and studied the binding of ROR1 and Cavin-1 using cell extract obtained by octyl glucoside.

When IP-WB analysis was conducted by the procedure of (3) above, it was clarified, as shown in FIGS. 4(1) and (2), that endogenous ROR1 and Cavin-1 bind since the two coprecipitated, but that ROR1 does not bind with Cavin-2 in NCI-H1975 and SK-LU-1, which are two different pulmonary adenocarcinoma cell lines. Similarly, when the binding of ROR1 and CAV1 was studied using cell extract obtained by octyl glucoside, as shown in FIGS. 4(3) and (4), it was clarified that endogenous ROR1 and CAV1 bind since the two coprecipitated in NCI-H1975 and SK-LU-1, which are two different pulmonary adenocarcinoma cell lines.

FIG. 4(5) is a photograph showing the results obtained when siROR1 was transfected into the pulmonary adenocarcinoma cell line NCI-H1975 cell line and the changes over time in the expression of CAV1 were analyzed by Western blotting together with the changes in the expression of ROR1. As shown in FIG. 4(5), it was judged that while silencing of ROR1 lowered the expression of ROR1 as soon as 24 hours after transfection of siROR1, downregulation of CAV1 was seen after 48 hours.

Therefore, IP-WB analysis was conducted of the influence on the binding of CAV1 and Cavin-1 under ROR1 silencing using cell extract from 24 hours after transfection of siROR1 when downregulation of ROR1 is seen and downregulation of CAV1 has not yet occurred. FIG. 4(6) is a photograph of the IP-WB analysis. As shown in FIG. 4(6), it was clarified that silencing of ROR1 decreases the binding of endogenous CAV1 and Cavin-1. These results showed the possibility that ROR1 binds with Cavin-1 and CAV1 and stabilizes the binding of Cavin-1 and CAV1 in pulmonary adenocarcinoma cell lines. CAV1 itself decomposes and normal caveolae cannot be constructed and formed since Cavin-1 cannot bind with CAV1 when Cavin-1 and CAV1 cannot bind with ROR1. It was also clarified that finding inhibitors that target the binding of ROR1 and Cavin-1 or CAV1, which are caveolar constituent factors, will lead to the identification of innovative inhibitors that trigger downregulation of CAV1, dysplasia of caveolae, and suppression of RTK activation.

### [Example 5]

### <Verification relating to the influence on each RTK activation via various ligand stimulations associated with ROR1 silencing in pulmonary adenocarcinoma cell lines>

It was clarified by the results of phospho-RTK array analysis in [Example 1] that silencing of ROR1 in pulmonary adenocarcinoma cell lines triggers lowering of the phosphorylation reaction (activation) of various RTKs in a steady state. In Example 5, how silencing of ROR1 influences the phosphorylation reaction by each ligand stimulation in these RTKs was analyzed by the procedure of (3) above similarly using the pulmonary adenocarcinoma cell line NCI-H1975. FIGS. 5(1)-(4) are photographs of Western blots when each ligand was used. As shown in FIGS. 5(1)-(4), it was clarified that silencing of ROR1 significantly lowers the phosphorylation reaction (activation) of RTKs (IGF-IR, insulinR, PDGFR) due to each ligand (IGF-I, IGF-II, insulin, PDGF). Although IGF-IR is activated by either IGF-I or IGF-II, silencing of ROR1 lowered the phosphorylation reaction of IGF-IR in either ligand stimulation.

### [Example 6]

### <Study of the importance of Cavin-1 in cell proliferation in pulmonary adenocarcinoma cell lines>

The influence of Cavin-1 on cell proliferation was studied by the procedure of (12) above. As shown in FIGS. 6(1) and (2), when silencing of ROR1, Cavin-1, and CAV1 was conducted in PC-9 and NCI-H1975, which are two different pulmonary adenocarcinoma cell lines, silencing of ROR1, Cavin-1, and CAV1 was understood to significantly inhibit cell proliferation in the two cell lines. Since silencing of Cavin-1 was also found to inhibit cell proliferation in the same way even when the different sequences siCavin-1#1 and #2 were used, it was understood that this is not a specific phenomenon based on a specific sequence of siCavin-1. There are reports that silencing of ROR1 inhibits cell proliferation (Patent Document 1 and Non-patent Document 5) and that silencing of CAV1 also inhibits cell proliferation (Non-patent Document 7), making reproducibility available, but it was newly understood from these results that silencing of Cavin-1 also inhibits the cell proliferation of pulmonary adenocarcinoma cell lines.

As mentioned above, it was shown that the construction and formation of caveolae become impossible in cancer cells since Cavin-1 cannot bind with CAV1 when binding of ROR1 with Cavin-1 or CAV1 becomes impossible. Factors other than ROR1 capable of suppressing the construction and formation of caveolae were therefore studied.

### [Example 7]

### <Verification relating to downregulation of CAV1 associated with IGF-IR silencing in pulmonary adenocarcinoma cell lines>

The influence of silencing of IGF-IR on expression of CAV1 was analyzed by Western blotting in various pulmonary adenocarcinoma cell lines by the procedure shown in (3) above. FIG. 7(1) shows the results when NCI-H1975 was used as the pulmonary adenocarcinoma cell line. Silencing of IGF-IR using the different sequences siIGF-IR#1 and #2 was found to downregulate CAV1 in all cases, in the same way as when treated by siROR1. These results clarified that downregulation of CAV1 is due to silencing of IGF-IR rather than a specific phenomenon based on a specific sequence of siIGF-IR. FIG. 7(2) shows the results when NCI-H358 was used as the pulmonary adenocarcinoma cell line. As in FIG. 7(1), downregulation of CAV1 was also found in the NCI-H358 cell line, in the same way as when treated by siROR1, when the expression of IGF-IR was suppressed using siIGF-IR#1 and #2. It is understood from these results that expression regulation of CAV1 by IGF-IR is observed even in different pulmonary adenocarcinoma cell lines, and it was clarified that IGF-IR, which is a factor other than ROR1, also induces downregulation of CAV1.

### [Example 8]

### <Binding of Cavin-1 and IGF-IR in pulmonary adenocarcinoma cell lines>

As in FIG. 8, when IP-WB analysis was conducted by the procedure of (3) above, it was clarified that endogenous Cavin-1 and IGF-IR bind since the two coprecipitate in NCI-H1975, which is a pulmonary adenocarcinoma cell line. It is understood from these results with Cavin-1 also binds with IGF-IR in addition to ROR1, and the importance of IGF-IR and Cavin-1 can also be considered in the same way as the importance of the binding of ROR1 and Cavin-1 since downregulation of CAV1 was found due to silencing of IGF-IR as well, in the same way as by silencing of ROR1.

### [Example 9]

### <Identification of the binding region of ROR1 by Cavin-1 and detection of binding of Cavin-1 and ROR1 for screening inhibitors to inhibit intracellular binding of the two>

The results of [Example 4] confirmed the binding of ROR1 and Cavin-1 in pulmonary adenocarcinoma cell lines. Next, various ROR1 kinase domain deletion mutants were prepared, as shown schematically in FIG. 9(1), by the procedure of (3) above to identify the binding site of ROR1 to which Cavin-1 binds. The various ROR1 kinase domain deletion mutants and Cavin-1 were expressed, and the binding with Cavin-1 and the binding site of ROR1 by Cavin-1 were identified by IP-WB analysis. FIG. 9(2) is a photograph of IP-WB analysis. IT was judged from the photograph of FIG. 9(2) that binding with Cavin-1 occurs in the region from amino acid 564 to amino acid 746 within the kinase domain of ROR1. These results show that ROR1 binds specifically to Cavin-1, and it is possible that binding of a region (region from amino acid 564 to amino acid 746) of a portion within the kinase domain of ROR1 and Cavin-1 stabilizes the expression of CAV1 and plays an important role in formation of normal caveolae and activation of various RTKs by caveolae.

It was judged from the results of [Example 3] above that the kinase activity of ROR1 is not necessary for expression regulation of CAV1 via ROR1 in pulmonary adenocarcinoma cell lines. The results of [Example 4] also clarified that ROR1 binds with Cavin-1 and CAV1 in pulmonary adenocarcinoma cell lines, and that ROR1 stabilizes the binding of Cavin-1 and CAV1. It was judged from these results that the binding of ROR1 with Cavin-1 and CAV1 is important in the formation of caveolae by ROR1 via CAV1 and in the subsequent activation of various RTKs. A binding detection system for screening that makes it possible to detect the intracellular protein-protein binding simply and easily was therefore constructed according to the procedure of (11) above. When the binding of the two was studied in HeLa cells using the intracellular region (ROR1-ICD) of ROR1 to which Cavin-1 binds and the full length (Cavin-1-WT) of Cavin-1 by image-based protein-protein interaction analysis that makes it possible to detect the binding of two proteins as intracellular fluorescent brightness, the formation of multiple foci was confirmed, as shown in the photograph analyzed using cell staining on the right in FIG. 9(3), and, significantly, their binding could be detected. On the other hand, as shown in the left-hand photograph in FIG. 9(3), virtually no foci were detected when a plasmid incorporating ROR1-ICD and a plasmid not incorporating Cavin-1 were expressed using the same vector as that which incorporated ROR1-ICD and Cavin-1-WT. It was therefore judged that the binding of ROR1-ICD and Cavin-1-WT has specificity.

Furthermore, when the binding of the two was studied using HeLa cells using the kinase inner regions (ROR1-TK2+TK3) of ROR1 to which Cavin-1 binds and the full length (Cavin-1-WT) of Cavin-1, the formation of multiple foci was confirmed, as shown by the photograph on the right in FIG. 9(4), and, significantly, their binding could be detected. On the other hand, as shown in the left-hand photograph in FIG. 9(4), virtually no foci were detected when a plasmid incorporating ROR1-TK2+TK3 and a plasmid not incorporating Cavin-1 were expressed using the same vector as that which incorporated ROR1-TK2+TK3 and Cavin-1-WT. It was therefore judged that the binding of ROR1-TK2+TK3 and Cavin-1-WT has specificity. Since these results were able to detect the binding of ROR1-ICD and ROR1-TK2+TK3 with Cavin-1-WT simply and easily on the intracellular level, low-molecular compounds that inhibit the binding of the two can be screened.

### [Example 10]

### <Identification of the binding region of ROR1 by CAV1>

Since it was possible to identify the binding region of ROR1 by Cavin-1 in Example 9, in order to identify the binding site of ROR1 by CAV1, various ROR1 deletion mutants were prepared, as shown schematically in FIG. 10(1), by the procedure of (3) above, and the binding of CAV1 and the binding site of ROR1 by CAV1 were identified by IP-WB analysis by expressing each of the ROR1 deletion mutants and CAV1. FIG. 10(2) is a photograph of IP-WB analysis. Based on FIG. 10(2), the region (serine-threonine-rich domain) from amino acid 853 to amino acid 876 of the intracellular region of ROR1 was judged to bind with CAV1. These results show that ROR1 binds specifically to CAV1, that the binding of part of the intracellular region of ROR1 (region from amino acid 853 to amino acid 876) and CAV1 stabilizes the expression of CAV1, in the same way as the binding of ROR1 and Cavin-1, and furthermore that this may play an important role in the formation of normal caveolae and the activation of various RTKs by the caveolae. A binding detection system for screening inhibitors that can detect protein-protein binding simply and easily intracellularly may be constructed by the same procedure as in Example 9.

### [Example 11]

### <Study of the importance of the cavin-1 binding region of ROR1 in CAV1 expression regulation>

As shown in Example 9, cavin-1 binds with the amino acid 564 to 746 region (TK2+TK3) within the kinase domain of the intracellular region of ROR1. To confirm that this binding region of ROR1 and cavin-1 is involved in the expression regulation of CAV1, mutants having an mutation to cancel the effect of siROR1 added to mutants from which the region of ROR1 that binds cavin-1 had been deleted were prepared by the procedure of (8) above, expressed in cells, and siROR1 treatment was conducted. FIG. 11 is a photograph showing the results of analysis by Western blotting. As shown in FIG. 11, although ROR1 was expressed, expression of CAV1 was lowered in cells transfected with ROR1-TKΔ2+TKΔ3m. It was understood from the above results that the region (amino acid 564 to 746 region) of ROR1 to which cavin-1 binds is essential for stabilizing the expression of CAV1. This result also suggests the importance of a screening system that inhibits the binding of this region of ROR1 and cavin-1, and allows us to infer that candidates for inhibitors that inhibit this binding will lower the expression of CAV1, that is, will inhibit cancer-specific caveolae formation.

### [Example 12] <Study of the importance of the CAV1 binding region of ROR1 in CAV1 expression regulation>

As shown in Example 10, CAV1 binds with the amino acid 853 to 876 region (S/T2) which is the serine-threonine-rich domain of the intracellular region of ROR1. To confirm that this binding region of ROR1 and CAV1 is involved in the expression regulation of CAV1, mutants having an mutation to cancel the effect of siROR1 added to mutants from which the region of ROR1 that binds CAV1 had been deleted were prepared by the procedure of (9) above, expressed in cells, and siROR1 treatment was conducted. FIG. 12 is a photograph showing the results of analysis by Western blotting. As shown in FIG. 12, although ROR1 was expressed, expression of CAV1 was lowered in cells transfected with ROR1-ΔS/T2m.

It was understood from the above results that the region (amino acid 853 to 876 region) of ROR1 to which CAV1 binds is essential for stabilizing the expression of CAV1, in the same way as the cavin-1 binding region. This result also suggests the importance of a screening system that inhibits the binding of this region of ROR1 and CAV1, and allows us to infer that candidates for inhibitors that inhibit this binding will lower the expression of CAV1, that is, will inhibit cancer-specific caveolae formation.

### [Example 13]

### <Study of the effect of silencing of ROR1 on cell proliferation and influence on the survival signal in EGFR-TKI-resistant cell lines under ligand stimulation>

The effects of silencing of ROR1 on cell proliferation and influence on the survival signal were evaluated by Western blotting and MTT assay in cell lines that had acquired EGFR-TKI resistance in the presence of various ligands by the procedure shown in (13) above.

Furthermore, the cell lines used here are vulvar squamous cell carcinoma cells and pulmonary adenocarcinoma cells which are regarded as clinically problematic cell lines. These cell lines are cell lines in which an EGFR inhibitor called EGFR-TKI is found to have an effect, but the effect of EGFR-TKI is completely canceled in the presence of various ligands such as IGF-I and HGF. For example, A431 cells, which are vulvar squamous cell carcinoma cells, are sensitive to gefitinib, but show resistance in the presence of IGF-I. PC-9 cells, which are pulmonary adenocarcinoma cells, are sensitive to gefitinib, but show resistance in the presence of HGF. Some lines of NCI-H1975 cells, which are pulmonary adenocarcinoma cells, show resistance to gefitinib (no effect is seen because they have a double mutation T790M of EGFR), but a second-generation EGFR-TKI has recently been developed, giving birth to CL-387, 785 as an irreversible EGFR-TKI, and causing the cells to show sensitivity. This has been put to clinical use. Another current problem is the resistance shown in the presence of this CL-387, 785 and HGF. The reason for the resistance in these cell lines is the activation of other RTKs (receptor tyrosine kinases) different from EGFR, such as IGF-IR and MET by IGF-I and HGF (bypass pathways).

The lower part of FIG. 13(1) is a graph showing the results of MTT assay in the presence of IGF-I and gefitinib when A431 was used as a vulvar squamous cell carcinoma cell line. Lowering of cell proliferation due to silencing of ROR1 was confirmed. The upper part of FIG. 13(1) is a photograph of a Western blot when A431 was used. Lowering of the phosphorylation of the AKT and IGF-IR involved in the survival signal by silencing of ROR1 was confirmed.

The lower part of FIG. 13(2) is a graph showing the results of MTT assay in the presence of HGF and gefitinib when PC-9 was used as a pulmonary adenocarcinoma cell line. Lowering of cell proliferation due to silencing of ROR1 was confirmed. The upper part of FIG. 13(2) is a photograph of a Western blot when PC-9 was used. Lowering of the phosphorylation of the AKT and MET involved in the survival signal by silencing of ROR1 was confirmed.

The lower part of FIG. 13(3) is a graph showing the results of MTT assay in the presence of HGF and CLL-387785 when NCI-H1975 was used as a pulmonary adenocarcinoma cell line. Lowering of cell proliferation due to silencing of ROR1 was confirmed. The upper part of FIG. 13(3) is a photograph of a Western blot when NCI-H1975 was used. Lowering of the phosphorylation of the AKT and MET involved in the survival signal was confirmed.

As shown in FIGS. 13(1)-(3), silencing of ROR1 was judged to trigger lowering of the survival signal (lowering of phosphorylation of AKT) and lowering of proliferation even in cell lines that had acquired EGFR-TKI resistance in the presence of various ligands (similarly, lowering of proliferation was confirmed with silencing of cavin-1 and CAV1 as well). In other words, since lowering of the phosphorylation reaction of IGF-IR and MET was found by Western blotting due to silencing of ROR1, silencing of ROR1 inhibits the formation of caveolae cancer-specifically, showing that the activation of RTKs present in the caveolae is suppressed. This result suggests the potential to in fact show a clinical effect even in patients having such drug resistance.

### INDUSTRIAL APPLICABILITY

The method for screening compounds that specifically suppress the formation of caveolae of cancer cells has the potential to suppress the binding of the Cavin-1 and CAV1 that participate in caveolae formation by a single compound and to be able to inhibit the activity of RTKs all at once. It is therefore possible to develop breakthrough drugs with few side effects. Patients for whom molecularly targeted drugs obtained by the above screening are indicated can be selected in advance by studying the combinations of expression of ROR1 and Cavin-1, ROR1 and CAV1, IGF-IR and Cavin-1, IGF-IR and CAV1, Cavin-1 and CAV1, or Cavin-1 in the cancer cells. This is therefore useful for cancer treatment with few side effects in medical institutions since a drug can be prescribed only for a patient in whom the molecularly targeted drug will have an effect.

[Sequence list]

## Claims

1. A method for screening a compound specifically suppressing the formation of caveolae of cancer cells,
wherein the method for screening includes
a step for bringing a test compound into contact with a system capable of detecting suppression of the binding of Cavin-1 and CAV1, and
a step for selecting a compound having activity to suppress the binding of Cavin-1 and CAV1.

2. The method for screening according to Claim 1, wherein the system capable of detecting suppression of the binding of Cavin-1 and CAV1 is a system capable of detecting suppression of the function of Cavin-1.

3. The method for screening according to Claim 2, wherein the system capable of detecting suppression of the function of Cavin-1 is a system capable of detecting suppression of the binding of ROR1 and Cavin-1 or a system capable of detecting suppression of the binding of IGF-IR and Cavin-1.

4. The method for screening according to Claim 1, wherein the system capable of detecting suppression of the binding of Cavin-1 and CAV1 is a system capable of detecting suppression of the function of CAV1.

5. The method for screening according to Claim 4, wherein the system capable of detecting suppression of the function of CAV1 is a system capable of detecting suppression of the binding of ROR1 and CAV1 or a system capable of detecting suppression of the binding of IGF-IR and CAV1.

6. A transformant into which have been introduced
a recombinant vector into which a nucleic acid that encodes an amino acid sequence including at least the amino acid region 564 to 746 within the amino acid sequence 428 to 937 of the intracellular region of human ROR1 has been inserted, and
a recombinant vector into which a nucleic acid that encodes the amino acid sequence of human Cavin-1 has been inserted.

7. A screening kit for a compound suppressing the binding of ROR1 and Cavin-1 including the transformant according to Claim 6.

8. A transformant into which have been introduced
a recombinant vector into which a nucleic acid that encodes an amino acid sequence including at least the amino acid region 853 to 876 within the amino acid sequence 428 to 937 of the intracellular region of human ROR1 has been inserted, and
a recombinant vector into which a nucleic acid that encodes the amino acid sequence of human CAV1 has been inserted.

9. A screening kit for a compound suppressing the binding of ROR1 and CAV1 including the transformant according to Claim 8.

10. A transformant into which have been introduced
a recombinant vector into which at least a nucleic acid that encodes an amino acid sequence including the amino acid region 564 to 746 and a nucleic acid that encodes an amino acid sequence of the amino acid region 853 to 876 within the amino acid sequence 428 to 937 of the intracellular region of human ROR1 have been inserted,
a recombinant vector into which a nucleic acid that encodes the amino acid sequence of human Cavin-1 has been inserted, and
a recombinant vector into which a nucleic acid that encodes the amino acid sequence of human CAV1 has been inserted.

11. A screening kit for a compound suppressing the binding of ROR1 and Cavin-1 and/or a compound suppressing the binding of ROR1 and CAV1 including the transformant according to Claim 10.

12. A recombinant vector into which at least a nucleic acid that encodes an amino acid sequence including the amino acid region 564 to 746 and/or a nucleic acid that encodes an amino acid sequence including the amino acid region 853 to 876 within the amino acid sequence 428 to 937 of the intracellular region of human ROR1 have been inserted.

13. A method for selecting a patient for whom a molecularly targeted drug is indicated, comprising
a step for acquiring cancer cells from the subject and
a step for measuring the expression of at least one selected from ROR1 and Cavin-1, ROR1 and CAV1, IGF-IR and Cavin-1, IGF-IR and CAV1, Cavin-1 and CAV1, Cavin-1, or CAV1 in the cancer cells.
